# EUROPÄISCHE PATENTANMELDUNG

(11) **EP 2 113 771 A2**
(43) Veröffentlichungstag der Anmeldung: **04.11.2009**
(21) Anmeldenummer: 09155922.9
(22) Anmeldetag: 23.03.2009
(51) Int. Cl.: G01N 33/00, G01N 30/84

(54) **Vorrichtung, Verwendung der Vorrichtung und Verfahren zur Herstellung eines flüssigen Geschmackstoffkondensates**

(30) Priorität: 30.04.2008 DE 102008022347
(71) Anmelder: Symrise GmbH & Co. KG, 37603 Holzminden (DE)
(72) Erfinder: Roloff, Michael, 34399 Oberweser-Arenborn (DE); Peter, Regina, 33034 Brakel-Istrup (DE); Lütkenhaus, Matthias, 37170 Uslar (DE)
(74) Vertreter: Stilkenböhmer, Uwe Michael

(57) **Zusammenfassung**

Die vorliegende Erfindung betrifft eine Vorrichtung, eine Verwendung der Vorrichtung und ein Verfahren zur Herstellung eines Geschmackstoffkondensates umfassend oder bestehend aus ein oder mehreren Geschmackstoffen, ein oder mehreren Referenzstoffen, Wasser und/oder Ethanol, sowie Verfahren zur sensorischen Bewertung des flüssigen Geschmackstoffkondensates.

## Beschreibung

### Gebiet der Erfindung

Die vorliegende Erfindung betrifft eine Vorrichtung, eine Verwendung der Vorrichtung und ein Verfahren zur Herstellung eines Geschmackstoffkondensates umfassend oder bestehend aus ein oder mehreren Geschmackstoffen, ein oder mehreren Referenzstoffen, Wasser und/oder Ethanol, sowie Verfahren zur sensorischen Bewertung des flüssigen Geschmackstoffkondensates.

### Stand der Technik

Aromen sind meistens komplexe Gemische einer Vielzahl von sensorisch wirksamen Substanzen, wie z.B. Pflanzenextrakte oder natürliche, naturidentische oder synthetische Aromastoffe. Aromakompositionen umfassend oder bestehend aus sensorisch wirksamen Substanzen enthalten zumindest zwei verschiedene Aromastoffe, die flüchtig (Geruchsstoffe) oder nichtflüchtig (Geschmacksstoffe) sein können. In der menschlichen Nase können flüchtige Geruchstoffe retronasal oder orthonasal wahrgenommen werden. Die geschmacklichen (gustatorischen) Eindrücke umami, salzig, sauer, süß und bitter werden durch Interaktion der Geschmacksstoffe mit den Geschmacksrezeptoren der Zunge wahrgenommen, weiterhin können hierbei auch weitere sensorische Eindrücke, beispielsweise prickelnde, kühlende, scharfe, stechende oder elektrisierende ("tingling") Effekte, wahrgenommen werden, die oftmals auf Grund von trigeminalen Reizungen entstehen. Der sensorische Gesamteindruck einer Aromakomposition wird grundsätzlich durch die Mengenverhältnisse der sensorisch wirksamen Stoffe einer Aromakomposition zueinander beeinflusst, wobei der sensorische Beitrag der einzelnen Aromastoffe entscheidend ist und nicht deren absolute Menge. In Lebensmitteln enthaltene sensorisch relevante Aromastoffe sind häufig nur in sehr geringen Mengen enthalten und vielfach noch nicht bekannt, obwohl durch diese Aromastoffe ein bedeutender geruchlicher und/oder geschmacklicher Beitrag, aufgrund ihrer hohen sensorischen Aktivität, geliefert wird. Ein wichtiges Bewertungskriterium ist daher der Aromawert, definiert als Quotient der Konzentration eines sensorisch wirksamen Aromastoffes zum geruchlichen beziehungsweise geschmacklichen Schwellenwert.

In der Aromenindustrie kommt heute eine Vielzahl unterschiedlicher sensorischer Bewertungsverfahren bzw. Vorrichtungen zur sensorischen Bewertung zum Einsatz. Die zur Bewertung verwendeten Proben werden hierbei oftmals sehr arbeits- und zeitintensiv vorbereitet. Leichtflüchtige Lösungsmittelanteile in Probenextrakten werden häufig durch Verwendung separater Verfahren reduziert. Probenmischungen werden durch zusätzliche Arbeitsschritte realisiert. Für den einzelnen Anwender ist es äußerst schwierig, bzw. nahezu unmöglich, die Bedienung der Vielzahl von verschiedenen Vorrichtungen und Verfahren zu beherrschen. Er beschränkt sich daher notgedrungen auf die bekannten und verfügbaren Vorrichtungen und Verfahren, was dazu führen kann, dass in bestimmten Fällen geeignete Vorrichtungen oder Verfahren nicht angewendet werden. In der Zeitschrift Chemie in unserer Zeit 2003, 37, 388-401, bzw. der darin zitierten Literatur, ist ein Übersichtsartikel zu diesem Thema veröffentlich worden.

Zur Geschmacks- und Geruchsstoffsuche sind bis heute keine High-throughput screening Verfahren zur sensorischen Bewertung der Prüfsubverbindungen bekannt, die zur schnellen Identifizierung neuer, sensorisch interessanter Verbindungen eingesetzt werden. Deshalb setzt die systematische Untersuchung eines Naturstoffextraktes heutzutage eine mehrstufige Trennung und Vorbehandlung zur Aufreinigung und Aufkonzentrierung der im Extrakt enthaltenen Einzelverbindungen voraus. Insbesondere die Probenvorbereitung zur sensorischen Bewertung in unterschiedlichen Testmedien nimmt hierbei sehr viel Zeit in Anspruch. Die Artefaktbildung, Instabilität oder Aufarbeitungsverluste bei der Isolierung/Vermischung von Geruch- und/oder Geschmackstoffen aus/mit komplexen Matrices kann aber auch zu geruchlichen bzw. geschmacklichen Fehlinformationen führen und stellt den Analytiker kontinuierlich vor neue Herausforderungen. Es sind deshalb eine Vielzahl von unterschiedlichen schonenden Extraktions-und Isolierungsverfahren entwickelt worden, die insbesondere individuell auf spezifische Aufgabenstellungen eingehen.

Zur flüssigchromatographischen Trennung von Aromakompositionen werden üblicherweise Elutionsmittel (mobile Phase) eingesetzt, die mit Wasser mischbar, jedoch gesundheitlich bedenklich oder giftig sind. Häufig verwendete Elutionsmittel sind Acetonitril, Methanol und Tetrahydrofuran sowie deren Mischungen mit wässrigen Puffern oder Wasser. Diese gesundheitlich bedenklichen Lösungsmittel müssen vor einer sensorischen Evaluierung entfernt werden. Durch Einsatz zeitintensiver, extraktiver oder thermischer Verfahren zur Entfernung der gesundheitlich bedenklicher Lösungsmittel kommt es häufig zu einer Änderung der Zusammensetzung der flüssigchromatographisch gewonnenen Fraktionen. Ursache hierfür sind thermische und/oder oxidative Einflüsse während der Behandlung. Die sensorischen Eigenschaften derartig behandelter Fraktionen unterscheiden sich häufig erheblich von den ursprünglichen Kompositionen. In der WO 2006/111476 ist bereits ein Verfahren zur Trennung von geruchlich und/oder geschmacklich wirksamen Komponenten mittels Hochtemperatur-Flüssigchromatographie (HTLC) sowie ein Verfahren zur direkten Verkostung der durch dieses Verfahren gewonnenen Fraktionen bekannt. Zwar erlaubt die Vorrichtung und das Verfahren gemäß WO 2006/111476 eine Auftrennung mehrerer in einer Geschmackstoffprobe enthaltenen Geschmackstoffe und die direkte zeitnahe ("on-line") bzw. synchrone Bestimmung des Aromawertes eines einzelnen Geschmackstoffes und damit seinen sensorischen Beitrag, jedoch ermöglicht die Vorrichtung und das Verfahren gemäß WO 2006/111476 nicht die sensorische Bewertung eines Geschmackstoffes hinsichtlich seiner verstärkenden (Synergieeigenschaft) bzw. seiner reduzierenden (Maskierungseigenschaft) Geschmackseigenschaften bezüglich weiterer in einer Aromakomposition enthaltenen Geschmackstoffe. Ein weiterer Nachteil an diesem Verfahren ist, dass das Eluat eines separierten Aromastoffes auf Grund seiner Verteilungsseigenschaften einen Ethanolgehalt von deutlich mehr als 15 Gew.-% bezogen auf das Gesamtgewicht des Eluats umfassen kann. Ein Ethanolgehalt von mehr als 15 Gew.-% bezogen auf das Gesamtgewicht des Eluats kann jedoch zum einen zu einer Beeinflussung der Geruchs- bzw. Geschmacksrezeptoren einer Testperson und damit zur Fehlinformation der sensorischen Eigenschaften führen und zum anderen kann ein Ethanolgehalt von mehr als 15 Gew.-% bei einer Verkostung von üblicherweise 5 ml pro Probe zu einer zeitweisen Bewusstseinsbeeinträchtigung der Testperson bei Verkostung mehrerer Proben führen, so dass lediglich wenige Verkostungen am Tag durchgeführt werden können.

In DE 3 941 533 A1 wird eine Schnittstelle zum Koppeln eines Flüssigkeitschromatographiesystems mit einem Fest- oder Gasphasendetektor beschrieben. Die erfindungsgemäße Schnittstelle umfasst einen Vernebler mit Thermospray- oder andere Sprühtechnik, gekoppelt an eine Verdampfungskammer und einen technischen Detektor, beispielsweise Massendetektor, wobei bereits im Vernebler die Temperatur auf mehr als 100° C erhöht wird, um besonders kleine Aerosoltröpfchen herzustellen und damit eine große Oberfläche zu erreichen, sodass im anschließenden Verdampfungsschritt fast das gesamte Lösungsmittel verdampft und ein "trockenes" Aerosol umfassend das Inertgas und die interessierenden Teilchen an den Gasphasen-oder Festphasen-Detektor zur Analyse weitergeleitet wird. Nachteilig an der Vorrichtung bzw. dem Verfahren gemäß DE 3 941 533 A1 ist, dass keine sensorische Bewertung durch eine Testperson hinsichtlich der geschmacklichen Eigenschaften einzelner Geschmackstoffe durchgeführt werden kann und insbesondere keine verstärkenden (Synergieeigenschaften) bzw. reduzierenden (Maskierungseigenschaften) eines einzelnen Geschmackstoffes bezüglich anderer in einer Aromakomposition enthaltenen Geschmackstoffe ermittelt werden können.

Es sind weiterhin Bewertungsverfahren durch elektronische Sensorsysteme ("*in vitro*") bekannt. Nachteilig hierbei ist die häufig fehlende Übertragbarkeit auf den Geschmacks- und/oder Geruchseindruck des Menschen.

Der vorliegenden Erfindung liegt deshalb die Aufgabe zu Grunde eine Vorrichtung und ein Verfahren zur sensorischen Bewertung von Geschmackstoffen bereitzustellen, die ein oder mehrere der folgenden Kriterien erfüllen:
- schnelle und einfache Herstellung einer Geschmackstoffzubereitung umfassend ein oder mehrere Geschmackstoffe zur direkten sensorischen Bewertung durch eine Testperson ("in vivo"), wobei die enthaltenen Geschmackstoffe bezüglich ihres geschmacklichen Eindruckes (im wesentlichen) nicht durch das Herstellverfahren beeinträchtigt werden,
- zeitnahe ("on-line") oder synchrone sensorischen Bewertung hinsichtlich des Geschmackes (Verkostung)
- sensorische Bewertung von verstärkenden (Synergieeigenschaften) bzw. reduzierenden (Maskierungseigenschaften) Geschmackseigenschaften eines Geschmackstoffes,
- Minimierung der Fehleinschätzung eines Geschmackseindruckes aufgrund der Beeinträchtigung der Geschmacksrezeptoren der Zunge durch den Ethanolgehalt und/oder
- Verkostung von 2, 3, 4, 5 oder mehreren Geschmackstoffproben an einem Tag ohne zeitweise Bewusstseinbeeinträchtigung für die Testperson aufgrund des Alkoholgehaltes.

### Zusammenfassung der Erfindung

Die Aufgabe der vorliegenden Erfindung wird durch die erfindungsgemäßen Gegenstände gemäß der unabhängigen Ansprüche gelöst.

Ein erster erfindungsgemäßer Gegenstand betrifft eine Vorrichtung (1) zur Herstellung eines flüssigen Geschmackstoffkondensats geeignet zur sensorischen Bewertung durch eine Testperson mit zwei Vernebeleinrichtungen (2, 2'), einer Mischkammer (3), einer Separationskammer (4) und einer Kondensationskammer (5), **dadurch gekennzeichnet, dass**
- die erste Vernebeleinrichtung (2) einen Flüssigkeitseinlass (21), einen Gaseinlass (22) und eine Verbindung zur Mischkammer (3) umfasst oder daraus besteht, so dass eine flüssige Geschmackstoffzubereitung umfassend ein oder mehrere Geschmackstoffe, Wasser und/oder Ethanol mittels des Flüssigkeitseinlasses (21) und ein Inertgas mittels des Gaseinlasses (22) in die Vernebeleinrichtung (2) zuführbar sind und die Vernebeleinrichtung (2) geeignet ist, die Flüssigkeit und das Inertgas zu einem Aerosol zu vernebeln und so in die Mischkammer (3) zuzuführen,
- die zweite Vernebeleinrichtung (2') einen Flüssigkeitseinlass (21'), einen Gaseinlass (22') und eine Verbindung zur Mischkammer (3) umfasst oder daraus besteht, so dass eine flüssige Referenzzubereitung umfassend ein oder mehrere Referenzstoffe, Wasser und/oder Ethanol mittels des Flüssigkeitseinlasses (21') und ein Inertgas mittels des Gaseinlasses (22') in die Vernebeleinrichtung (2') zuführbar sind und die Vernebeleinrichtung (2') geeignet ist, die Flüssigkeit und das Inertgas zu einem Aerosol zu vernebeln und so in die Mischkammer (3) zuzuführen,
- die Mischkammer (3) eine Verdampfungseinrichtung (31) und eine Verbindung zur Separationskammer (4) umfasst oder daraus besteht, wobei die Mischkammer (3) nach der ersten und zweiten Vernebeleinrichtung (2,2') angeordnet und so damit verbunden ist, dass die mittels der ersten und zweiten Vernebeleinrichtung (2,2') gebildeten Aerosole in die Mischkammer (3) separat zuführbar sind und die Verdampfungseinrichtung (31) geeignet ist, einen Teil oder das Ethanol der zugeführten Aerosole teilweise oder vollständig in die Gasphase zu überführen,
- die Separationskammer (4) einen Gasauslass (41) und eine Verbindung zur Kondensationskammer (5) umfasst oder daraus besteht, wobei die Separationskammer (4) nach der Mischkammer (3) angeordnet und so damit verbunden ist, dass das in die Gasphase überführte Ethanol und das verbleibende Aerosol in die Separationskammer (4) zuführbar sind und ein Teil oder die Ethanol-Gasphase aus der Vorrichtung (1) durch den Gasauslass (41) entfernbar ist und
- die Kondensationskammer (5) einen Flüssigkeitsauslass (51) und eine Kondensationseinrichtung (52) umfasst oder daraus besteht, wobei die Kondensationskammer (5) nach der Separationskammer (4) angeordnet und so damit verbunden ist, dass das in der Separationskammer (4) nach teilweiser oder vollständiger Entfernung der Ethanol-Gasphase verbleibende Aerosol in die Kondensationskammer (5) zuführbar ist und die Kondensationseinrichtung (5) geeignet ist, das zugeführte Aerosol ganz oder teilweise zu kondensieren und das Kondensat aus der Kondensationskammer (5) durch den Flüssigkeitsauslass (51) entfernbar ist.

Ein zweiter erfindungsgemäßer Gegenstand betrifft ein Verfahren zur Herstellung eines flüssigen Geschmackstoffkondensats geeignet zur sensorischen Bewertung von verstärkenden oder reduzierenden Geschmackstoffeigenschaften durch eine Testperson umfassend oder bestehend aus den folgenden Schritten:
a) Bereitstellung einer flüssigen Geschmackstoffzubereitung, die ein oder mehrere Geschmackstoffe, Wasser und/oder Ethanol umfasst oder daraus besteht, sowie eines Inertgases,
b) Bereitstellung einer flüssigen Referenzzubereitung, die ein oder mehrere Referenzstoffe, Wasser und/oder Ethanol umfasst oder daraus besteht, sowie eines Inertgases,
c) Zuführung der flüssigen Geschmackstoffzubereitung aus Schritt a) durch einen Flüssigkeitseinlass (21) und des Inertgases durch einen Gaseinlass (22) in eine erste Vernebeleinrichtung (2) mittels der die Geschmackstoffzubereitung und das Inertgas zu einem Aerosol vernebelt und so einer Mischkammer (3) zugeführt werden,
d) Zuführung der flüssigen Referenzzubereitung aus Schritt b) durch einen Flüssigkeitseinlass (21') und des Inertgases durch einen Gaseinlass (22') in eine zweite Vernebeleinrichtung (2') mittels der die Referenzzubereitung und das Inertgas zu einem Aerosol vernebelt und so einer Mischkammer (3) zugeführt werden,
e) Überführung eines Teils des oder des gesamten Ethanolanteils der in Schritt d) in die Mischkammer (3) zugeführten Aerosole mittels einer Verdampfungseinrichtung (31) in eine Gasphase,
f) Weiterleitung des in Schritt e) gebildeten Aerosols in eine mit der Mischkammer (3) verbundenen Separationskammer (4) mit Gasauslass (41) und Entfernung eines Teils des oder des gesamten gasförmigen Ethanolanteils aus der Separationskammer (4) durch den Gasauslass (41),
g) Weiterleitung des in Schritt f) gebildeten Aerosols in eine mit der Separationskammer (4) verbundene Kondensationskammer (5) mit Flüssigkeitsauslass (51) und Kondensationseinrichtung (52) und Kondensation des Aerosols mittels der Kondensationseinrichtung (52) zu einem Geschmackstoffkondensat umfassend oder bestehend aus dem oder den Geschmackstoffen, dem oder den Referenzstoffen, Wasser und/oder Ethanol und
h) Entfernung des in Schritt g) gebildeten Geschmackstoffkondensats aus der Kondensationskammer (5) durch den Flüssigkeitsauslass (51).

Ein dritter erfindungsgemäßer Gegenstand betrifft ein Verfahren zur sensorischen Bewertung von verstärkenden und/oder reduzierenden Geschmackseindrücken ein oder mehrerer Geschmackstoffe umfassend oder bestehend aus den folgenden Schritten:
i. Herstellung eines flüssigen Geschmackstoffkondensats umfassend oder bestehend aus ein oder mehreren Geschmackstoffen, ein oder mehreren Referenzstoffen, Wasser und/oder Ethanol gemäß des vorstehenden erfindungsgemäßen Verfahrens und
ii. Zuführung des Geschmackstoffkondensates in den Mund einer Testperson zur sensorischen Bewertung von verstärkenden und/oder reduzierenden Geschmackseindrücken des oder der Geschmackstoffe in dem Geschmackstoffkondensat.

Ein vierter erfindungsgemäßer Gegenstand betrifft eine Verwendung einer erfindungsgemäßen Vorrichtung zur Herstellung eines flüssigen Geschmackstoffkondensates umfassend oder bestehend aus ein oder mehreren Geschmackstoffen, ein oder mehreren Referenzstoffen, Wasser und/oder Ethanol.

Ein fünfter erfindungsgemäßer Gegenstand betrifft ein Verfahren zur Verdünnung einer flüssigen Geschmackstoffzubereitung umfassend oder bestehend aus den folgenden Schritten:
a) Bereitstellung einer flüssigen Geschmackstoffzubereitung, die ein oder mehrere Geschmackstoffe, Wasser und Ethanol mit einem Ethanolgehalt von > 15 Gew.-% bezogen auf das Gesamtgewicht der Geschmackstoffzubereitung umfasst oder daraus besteht, sowie eines Inertgases,
b) Bereitstellung einer Verdünnungslösung, die Wasser und/oder Ethanol umfasst oder daraus besteht, wobei der Ethanolgehalt ≤ 15 Gew.-% bezogen auf das Gesamtgewicht der Lösung beträgt, sowie eines Inertgases,
c) Zuführung der flüssigen Geschmackstoffzubereitung aus Schritt a) durch einen Flüssigkeitseinlass (21) und des Inertgases durch einen Gaseinlass (22) in eine erste Vernebeleinrichtung (2) mittels der die Geschmackstoffzubereitung und das Inertgas zu einem Aerosol vernebelt und so einer Mischkammer (3) zugeführt werden,
d) Zuführung der Verdünnungslösung aus Schritt b) durch einen Flüssigkeitseinlass (21') und des Inertgases durch einen Gaseinlass (22') in eine zweite Vernebeleinrichtung (2') mittels der die Verdünnungslösung und das Inertgas zu einem Aerosol vernebelt und so der Mischkammer (3) zugeführt werden,
e) Überführung eines Teils des oder des gesamten Ethanolanteils der in Schritt d) in die Mischkammer (3) zugeführten Aerosole mittels einer Verdampfungseinrichtung (31) in eine Gasphase,
f) Weiterleitung des in Schritt e) gebildeten Aerosols in eine mit der Mischkammer (3) verbundenen Separationskammer (4) mit Gasauslass (41) und Entfernung eines Teils des oder des gesamten gasförmigen Ethanolanteils aus der Separationskammer (4) durch den Gasauslass (41),
g) Weiterleitung des in Schritt f) gebildeten Aerosols in eine mit der Separationskammer (4) verbundene Kondensationskammer (5) mit Flüssigkeitsauslass (51) und Kondensationseinrichtung (52) und Kondensation des Aerosols mittels der Kondensationseinrichtung (52) zu einem Geschmackstoffkondensat umfassend oder bestehend aus dem oder den Geschmackstoffen, Wasser und Ethanol, wobei der Ethanolgehalt ≤ 15 Gew.-% bezogen auf das Gesamtgewicht des Kondensates beträgt und
h) Entfernung des in Schritt g) gebildeten Geschmackstoffkondensats aus der Kondensationskammer (5) durch den Flüssigkeitsauslass (51).

Ein sechster erfindungsgemäßer Gegenstand betrifft ein Verfahren zur sensorischen Bewertung eines oder mehrerer Geschmackstoffe umfassend oder bestehend aus den folgenden Schritten:
i. Herstellung eines flüssigen Geschmackstoffkondensats umfassend oder bestehend aus ein oder mehreren Geschmackstoffen, Wasser und Ethanol gemäß dem fünften Erfindungsgegenstandes, wobei der Ethanolgehalt ≤ 15 Gew.-% bezogen auf das Gesamtgewicht des Kondensates beträgt, und
ii. Zuführung des Geschmackstoffkondensates aus Schritt i) in den Mund einer Testperson zur sensorischen Bewertung des oder der Geschmackstoffe.

Ein siebter erfindungsgemäßer Gegenstand betrifft eine Verwendung einer erfindungsgemäßen Vorrichtung zur Herstellung eines flüssigen Geschmackstoffkondensates umfassend oder bestehend aus ein oder mehreren Geschmackstoffen, Wasser und Ethanol, wobei der Ethanolgehalt ≤ 15 Gew.-% bezogen auf das Gesamtgewicht des Kondensates beträgt.

Die Gewichtsprozentanteile (Gew.-%) in Bezug auf die erfindungsgemäßen Gegenstände sind jeweils auf das Gesamtgewicht der jeweiligen flüssigen Zubereitung bezogen.

Die erfindungsgemäßen Gegenstände umfassen ebenfalls in jeder denkbaren Kombination die bevorzugten Ausgestaltungen, wie sie in der nachfolgenden Beschreibung, den Figuren, den Ausführungsbeispielen und abhängigen Ansprüchen dargestellt werden.

### Figurenbeschreibung:

Die Figuren zeigen sowohl einen schematischen Aufbau einer erfindungsgemäßen Vorrichtung (1) als auch die erfindungsgemäße Vorrichtung (1) in Verbindung mit vor- und nachgeschalteten Systemen.

### Von den Figuren zeigt:

- Fig. 1:: einen schematischen Aufbau einer erfindungsgemäßen Vorrichtung (1).
- Fig. 2:: einen schematischen Aufbau einer erfindungsgemäßen Vorrichtung (1) gekoppelt an ein Flüssigchromatographiesystem (6).
- Fig. 3:: einen schematischen Aufbau einer erfindungsgemäßen Vorrichtung (1) gekoppelt an ein Probengefäß (61) enthaltend eine Geschmackstoffprobe.

### Bezugszeichenliste:

- (1): erfindungsgemäße Vorrichtung
- (2): Erste Vernebeleinrichtung
- (21): Flüssigkeitseinlass der ersten Vernebeleinrichtung
- (22): Gaseinlass der ersten Vernebeleinrichtung
- (2'): Zweite Vernebeleinrichtung
- (21'): Flüssigkeitseinlass der zweiten Vernebeleinrichtung
- (22'): Gaseinlass der zweiten Vernebeleinrichtung
- (3): Mischkammer
- (31): Verdampfungseinrichtung
- (32): Innere Wand der Mischkammer
- (33): Äußere Wand der Mischkammer
- (34): Einlass für ein Medium zur Temperaturregelung
- (35): Auslass für ein Medium zur Temperaturregelung
- (4): Separationskammer
- (41): Gasauslass
- (411): Ende des Gasauslass im unteren Bereich innerhalb der Separationskammer
- (412): Ende des Gasauslass im Bereich außerhalb der Separationskammer/Vorrichtung
- (42): Verwirbelungseinrichtung
- (43): Wand der Separationskammer
- (5): Kondensationskammer
- (51): Flüssigkeitsauslass
- (52): Kondensationseinrichtung
- (53): Innere Wand der Mischkammer
- (54): Äußere Wand der Mischkammer
- (55): Einlass für ein Medium zur Temperaturregelung
- (56): Auslass für ein Medium zu Temperaturregelung
- (6): Flüssigkeitschromatographiesystem
- (61): Probengefäß mit Geschmackstoffprobe
- (62): Pumpe
- (7): Inertgasbehälter
- (8): Detektoreinrichtung

### Detaillierte Beschreibung der Erfindung

Der vorliegenden Erfindung liegt die Erkenntnis zu Grunde, dass mit der erfindungsgemäßen Vorrichtung ein flüssiges Geschmackstoffkondensat umfassend oder bestehend aus ein oder mehreren Geschmackstoffen, ein oder mehreren Referenzstoffen, Wasser in einer einfachen und schnellen Weise hergestellt werden kann und so (i) eine zeitnahe ("on-line") oder synchrone sensorische Bewertung hinsichtlich Synergie- und/oder Maskierungseigenschaften durch eine Testperson ("in vivo") erlaubt. Das erfindungsgemäß herstellbare Geschmackskonzentrat gemäß des fünften bis siebten erfindungsgemäßen Gegenstandes weist zudem bei einer in vivo sensorischen Bewertung ein geringes Risiko für eine Fehleinschätzung der sensorischen Eigenschaften des oder der Geschmacksstoffe auf, da aufgrund des Ethanolgehaltes von ≤ 15 Gew.% bezogen auf das Gesamtgewicht des Geschmackstoffkondensats die Geschmacksrezeptoren nicht (wesentlich) beeinträchtigt werden und es zudem so ermöglicht wird, dass das Bewusstsein der Testperson für mehr als zwei Proben am Tag nicht zeitweise beeinträchtigt wird.

Im Sinne der vorliegenden Erfindung umfasst der Begriff "Geschmackstoff" eine sensorisch wirksame Substanz, die einen Geschmackseindruck hervorruft, d.h. selbst vermittelt oder die geschmackliche Wahrnehmung einer anderen Substanz auf Grund eines Eigengeschmacks verändert, d.h. modifiziert oder verstärkt, wobei die Substanz einen Dampfdruck < 0,05 Pa bei 25° C aufweist.

Im Sinne der vorliegenden Erfindung bedeutet der Begriff "Geschmackstoffzubereitung" eine flüssige Zubreitung umfassend oder bestehend aus ein oder mehreren Geschmackstoffen, Wasser und/oder Ethanol. Bevorzugt hat die erfindungsgemäße Geschmackstoffzubereitung einen Ethanolgehalt von > 15 Gew.-%, weiter bevorzugt > 20 Gew.-% bezogen auf das Gesamtgewicht der Geschmackstoffzubereitung. Üblicherweise beträgt der Geschmackstoffanteil ≤ 5 Gew.-%, bevorzugt ≤ 2,5 Gew.-%, weiter bevorzugt ≤ 1 Gew.-% und mindestens 10⁻¹² Gew.-% und der Anteil der Wasser-Ethanol-Mischung beträgt üblicherweise größer oder gleich 95 Gew.-%, bevorzugt größer oder gleich 97,5 Gew.-%, weiter bevorzugt größer oder gleich 99 Gew.-% jeweils bezogen auf das Gesamtgewicht der Geschmackstoffzubereitung. Der Ethanolgehalt in der Wasser-Ethanol-Mischung liegt vorzugsweise im Bereich von 16 bis 95 Gew.-%, weiter bevorzugt im Bereich von 25 bis 90 Gew.-%, besonders bevorzugt im Bereich von 40 bis 85 Gew.-%, ganz besonders bevorzugt im Bereich von 50 bis 80 Gew.-%, jeweils bezogen auf das Gesamtgewicht der Wasser-Ethanol-Mischung. Je höher der Anteil an Ethanol in der Wasser-Ethanol-Mischung der Geschmackstoffzubereitung ist, desto weniger fein muss die Geschmackstoffzubereitung, bei vergleichbarer Temperatur, vernebelt werden, um eine erfindungsgemäße Reduzierung des Ethanolgehaltes zu erhalten.

Der Wasser- und/oder Ethanolanteil der erfindungsgemäßen Geschmackstoffzubereitung kann zusätzlich einen oder mehrere Bestandteile umfassen oder daraus bestehen, die ausgewählt werden aus der Gruppe bestehend aus Propylenglykol, Glycerin, Triacetin (Glycerintriacetat), physiologisch verträglichen Salzen (beispielsweise Natriumchlorid), physiologisch verträglichen Säuren (beispielsweise Phosphorsäure, Essigsäure), physiologisch verträglichen Puffersubstanzen (beispielsweise Natriumphosphate, Natriumacetat), Öle und Fette, wobei die Konzentration des oder der Bestandteile in der Wasser-Ethanol-Mischung zweckmäßigerweise jeweils so gewählt wird, dass die erfindungsgemäße Geschmackstoffzubereitung als Ganzes gesundheitlich unbedenklich bleibt.

Sofern der Wasser- und/oder Ethanolanteil der erfindungsgemäßen Geschmackstoffzubereitung physiologisch verträgliche Salze (beispielsweise Natriumchlorid), physiologisch verträgliche Säuren (beispielsweise Phosphorsäure, Essigsäure) und/oder physiologisch verträgliche Puffersubstanzen (beispielsweise Natriumphosphate, Natriumacetat) umfasst, liegt der Gesamtanteil dieser Salze, Säuren und/oder Puffersubstanzen vorzugsweise bei ≤ 10 Gew.-%, bevorzugt bei ≤ 5 Gew.-%, besonders bevorzugt im Bereich von 0,001 bis 2 Gew.-%, jeweils bezogen auf das Gesamtgewicht der Geschmackstoffzubereitung.

Im Sinne der vorliegenden Erfindung bedeutet der Begriff "Referenzstoff" eine sensorisch wirksame Substanz, die gezielt in einem erfindungsgemäßen Verfahren verwendet wird, um einen Geschmackseindruck aus der Gruppe umami, salzig, sauer, süß, bitter, oder prickelnde, kühlende, scharfe, stechende oder elektrisierende ("tingling") hervorzurufen, so dass eine sensorische Bewertung von Synergie- bzw. Maskierungseigenschaften der zu testenden Geschmackstoffe aus der Geschmackstoffzubereitung vorgenommen werden kann. Bevorzugt werden die erfindungsgemäß zu verwendenden Referenzstoffe ausgewählt aus der Gruppe bestehend aus:

| | |
|---|---|
| umami | Natriumglutamat, Inosin 5'-monophosphat, Cyclopropylcarbonsäure-N-neomenthylamid, N-Ethyl-2-isopropyl-5-methylbicyco[4.1.0]heptan-7-carbonsäureamid, N-Cyclopentyl-2-isopropyl-5-methylbicyco[4.1.0]heptan-7-carbonsäureamid, bevorzugt Natriumglutamat |
| salzig | Natriumchlorid, Lithiumchlorid, Kaliumchlorid, bevorzugt Natriumchlorid |
| sauer | Citronensäure, Milchsäure, Essigsäure |
| Süß | Glucose, Fructose, Saccharose, Sorbitol, Mannitol, Erythritol, Tagatose, Aspartam, Saccharin und dessen Salze, Sucralose, Neotam, Neohesperidindihydrochalcon, Acesulfam-Kalium, Cyclamat, Natrium-Cyclamat, |
| | Thaumatin, Stevioside, Rebaudioside, bevorzugt Glucose oder Saccharose |
| bitter | Coffein, Theobromin, Amarogentin, Chinin und dessen Salze, Humulone, Isoalphasäuren, Catechine, insbesondere Teecatechine, bittere Peptide wie z.B. L-Leu-L-Trp-OH, Kaliumsalze, Magnesiumsulfat, Salicin, bevorzugt Coffein oder Chinin |
| prickelnd/tingling | Spilanthol, Pellitorin, trans- Pellitorin, Sanshoole und Hydroxysanshoole |
| kühlende | Menthol, I-Menthol; Icilin; Menthancarbonsäureamide, bevorzugt L-Menthancarbonsäure-N-ethylamid (WS-3), *N*^{α}-(L-Menthancarbonyl)-glycinethylester (WS-5), 2,3-Dimethyl-2-(2-propyl)-butansäure-N-methylamid (WS-23); Menthylacetat; L-Menthyllactat; L-Menthylethylenglycolcarbonat; L-Menthylpropylenglycolcarbonat; Menthylacetoacetat; Menthyl-3-hydroxybutyratM; Menthonglycerinketal; und Isopulegol |
| scharfe | Capsaicin; Dihydrocapsaicin; Piperin; Gingerole, bevorzugt [6]-Gingerol; Paradole, bevorzugt [6]-Paradol; Shogaole; Rubemamin; Nonivamid; Vanillomandelsäurealkylamide (insbesondere die heptyl-, octyl-, nonylamide), Drimane, beispielsweise Polygodial |
| stechende | Isothiocyanate, bevorzugt Phenylethylisothiocyanat, Allylisothiocyanat, Isobutly- oder Isopropylisothiocyanat |

Im Sinne der vorliegenden Erfindung bedeutet der Begriff "Referenzzubereitung" eine flüssige Zubreitung umfassend oder bestehend (a) aus ein oder mehreren erfindungsgemäß zu verwendenden Referenzstoffen und (b) Wasser und/oder Ethanol.

Im Sinne der vorliegenden Erfindung wird das "Geschmackstoffkondensat" aus der erfindungsgemäßen Geschmackstoffzubereitung hergestellt und umfasst oder besteht aus ein oder mehreren Geschmackstoffen, ein oder mehrere Referenzstoffe, Wasser und Ethanol. In einer bevorzugten Ausgestaltung weist das erfindungsgemäße Geschmackstoffkondensat einen Ethanolgehalt von ≤ 15 Gew.-%, weiter bevorzugt im Bereich von 0 bis 10 Gew.-%, besonders bevorzugt im Bereich von 0,1 bis 8 Gew.-%, ganz besonders bevorzugt im Bereich von 1 bis 5 Gew.-%, jeweils bezogen auf das Gesamtgewicht des Geschmackstoffkondensates auf. Dementsprechend liegt in der bevorzugten Ausgestaltung das erfindungsgemäße Geschmackstoffkonzentrat im Vergleich zur erfindungsgemäßen Geschmackstoffzubereitung in Bezug auf den Ethanolgehalt reduziert vor, wodurch eine Gefahr von Fehleinschätzungen hinsichtlich des Geschmackes oder von zeitweisen Bewusstseinsbeeinträchtigung einer Testperson, die auf Grund eines Ethanolgehaltes von mehr als 15 Gew.-% resultieren kann, minimiert wird. Desweiteren können die zusätzlichen Bestandteile der Geschmackstoffzubereitung ebenfalls in dem erfindungsgemäßen Geschmackstoffkonzentrat vorliegen.

Im Sinne der vorliegenden Erfindung bedeutet der Begriff "Inertgas", dass das zugeführte Inertgas die erfindungsgemäße "flüssigen Zubereitung", insbesondere die erfindungsgemäße "Geschmackstoffzubereitung" nicht in chemischer Hinsicht verändert. Inertgase werden bevorzugt ausgewählt aus der Gruppe bestehend aus: Stickstoff, Helium oder Argon. Besonders bevorzugt wird Stickstoff als Inertgas erfindungsgemäß verwendet.

Im Sinne der vorliegenden Erfindung bedeutet der Begriff "sensorische Bewertung", dass eine Probe, üblicherweise ≤ 5 ml, eines erfindungsgemäß hergestellten Geschmackstoffkondensates durch eine Testperson in den Mund genommen und gegebenenfalls geschluckt wird, wobei die Probe gustatorsich und/oder retronasal auf sensorischen Eindrücke, beispielsweise umami, salzig, sauer, süß, bitter, oder prickelnde, kühlende, scharfe, stechende oder elektrisierende ("tingling") Effekte, bewertet wird. Insbesondere werden auch verstärkende oder maskierende Eigenschaften der zu testenden Geschmackstoffe gegenüber dem oder den eingesetzten Referenzstoffen bewertet.

Im Sinne der vorliegenden Erfindung ist ein erfindungsgemäß zu verwendendes "Flüssigkeitschromatographiesystem (6)" eine Vorrichtung, die zur Durchführung einer Flüssigkeitschromatographie [liquid chromatography (LC-Chromatographie), bevorzugt high pressure liquid chromatography (HPLC-Chromatographie) und insbesondere high temperature liquid chromatography (HTLC-Chromatographie)] einer Geschmackstoffzubereitung mit Detektion des oder der Geschmackstoffe geeignet ist.

Geeignete Trenntechniken für die erfindungsgemäß zu verwendenden Flüssigkeitschromatographiesysteme (6) umfassen: Reversed-Phase Chromatographie (RP), Normal-Phase Chromatographie (NP), Größenausschluß-Chromatographie (SEC) und Hydrophile Interaktionschromatographie (HILIC).

Üblicherweise werden die Detektoren ausgewählt aus der Gruppe bestehend aus Massenselektiven Detektoren (MS), Ultraviolett Detektoren (UV), Diodenarray Detektoren (DAD), Brechungsindex-Detektoren (RI), Verdampfungslichtstreu Detektoren (ELSD - evaporative light scattering detector), Fluoreszenz Detektoren (FLD) oder Charged Aerosol Detektoren (CAD).

Bevorzugt umfasst oder besteht ein HPLC-Flüssigkeitschromatographiesystem (6) aus einem Degasser (beispielsweise 3315, Fa. ERC), zwei Pumpen (beispielsweise Sun Flow 100, Fa. Sunchrom), einem Mischer (beispielsweise dynamic/static Mixer, Fa. Sunchrom), einem Autosampler (beispielsweise Midas, Fa. Spark), einer Chromatographiesäule (beispielsweise einer Hamilton PRP-1 250x10mm Säule) und einem Detektor (bevorzugt einem Diodenarray-Detector, beispielsweise Spectra Flow 600, Fa. Sunchrom). Bei einem erfindungsgemäß zu verwendenden HTLC-Flüssigkeitschromatographiesystem ist die Chromatographiesäule üblicherweise in einem Säulenofen geeignet zur Einstellung der benötigten Temperatur in der Säule (beispielsweise Polaratherm, Fa. Selerity) angeordnet.

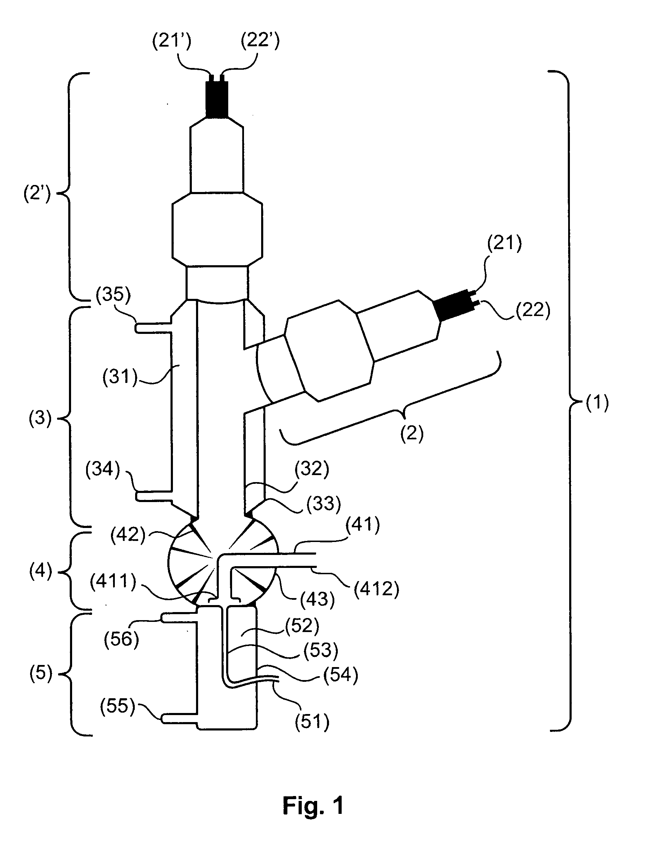

**Figur 1** zeigt einen schematischen Aufbau (im Maßstab 1 : 2, d.h. die Vorrichtung ist doppelt so groß wie die Abbildung in Figur 1) einer erfindungsgemäßen Vorrichtung (1), die zur sensorischen Bewertung einer erfindungsgemäßen Geschmackstoffzubereitung, geeignet ist.

Die flüssige Geschmackstoffzubereitung wird durch den Flüssigkeitseinlass (21) in die erste Vernebeleinrichtung (2) zugeführt. Zusätzlich wird ein Inertgas durch den Gaseinlass (22) (im wesentlichen) gleichzeitig zur Flüssigkeitseinleitung in die Vernebeleinrichtung (2) zugeführt. Mittels der Vernebeleinrichtung (2) wird die zugeführte Flüssigkeit und das zugeführte Inertgas zu einem Aerosol vernebelt und in die Mischkammer (3) als Aerosol zugeführt.

Die flüssige Referenzzubereitung wird durch den Flüssigkeitseinlass (21') in die zweite Vernebeleinrichtung (2') zugeführt. Zusätzlich wird ein Inertgas durch den Gaseinlass (22') (im wesentlichen) gleichzeitig zur Flüssigkeitseinleitung in die Vernebeleinrichtung (2') zugeführt. Mittels der Vernebeleinrichtung (2') wird die zugeführte Flüssigkeit und das zugeführte Inertgas zu einem Aerosol vernebelt und in die Mischkammer (3) als Aerosol zugeführt.

Die flüssige Referenzzubereitung kann kontinuierlich der zweiten Vernebeleinrichtung (2') zugeführt werden, so dass der Referenzstoff die gesamte Zeit im Geschmackstoffkondensat umfasst ist. Für den Fall, dass die Geschmackstoffzubereitung eine Eluat einer LC-Chromatographie ist und das Eluat nicht den zu testenden Geschmackstoff umfasst, kann dann der Referenzstoff in dem Kondensat gleichzeitig als Vergleichswert für den sensorischen Eindruck des Referenzstoffes dienen.

In einer alternativen Ausgestaltung kann die Zufuhr der Referenzzubereitung und/oder die Gaszufuhr zur zweiten Vernebeleinrichtung (2') mittels Regelungen gestoppt werden. Bevorzugt ist die Gaszufuhr zur zweiten Vernebeleinrichtung (2') geöffnet. Gemäß dieser Ausgestaltung wird die sensorische Bewertung hinsichtlich des sensorischen Beitrags des oder der zu testenden Geschmackstoffe ermöglicht.

In einer bevorzugten Ausgestaltung wird die erste und/oder zweite Vernebeleinrichtung (2,2') bei einer Temperatur ≤ 100 °C, vorzugsweise ≤ 75 °C, weiter bevorzugt in einem Bereich von 10 bis 40 °C, besonders bevorzugt in einem Bereich von 15 bis 30 °C und ganz besonders bevorzugt in einem Bereich von 20 bis 25 °C betrieben. Diese Temperaturbereiche minimieren das Risiko von Siedeverzügen. Zudem kann man über die Einstellung der Temperatur die Tröpfchengröße des Aerosols regeln. Bei Tröpfchen des Aerosols mit einem durchschnittlich kleineren Durchmesser ist die Oberfläche der Flüssigkeit im Aerosol größer als bei Tröpfchen mit einem durchschnittlich größeren Durchmesser. Je größer die Oberfläche der Tröpfchen im Aerosol, desto höher wird der Anteil der Verdampfung des Ethanols bzw. des Ethanol-WasserGemisches im Aerosol im anschließenden Verdampfungsschritt sein.

Die zugeführte Geschmackstoffzubereitung und Referenzzubereitung und das zugeführte Inertgas werden mit einer üblichen Fließgeschwindigkeit bzw. einem üblichen Druck in die erste und zweite Vernebeleinrichtung (2,2') zugeführt, damit einerseits die Vernebelung zu einem Aerosol ermöglicht wird und andererseits das gebildete Aerosol durch die erfindungsgemäße Vorrichtung (1) transportiert werden kann. Fließgeschwindigkeiten und Drücke können von der Ausgestaltung der ersten und zweiten Vernebeleinrichtung (2') abhängen und dementsprechend variieren.

Erfindungsgemäß zu verwendende Vernebeleinrichtungen (2,2') sind bevorzugt Vernebeleinrichtungen für Verdampfungslichtstreudetektoren (beispielsweise Evaporative Light Scattering Detector, ELSD), die beispielsweise in US 7,290,723, US 6,528018, US 6,485,689, US 6,362,880, US 6,229,605, US 6,151,113 und US 6,122,055 dargestellt werden, wobei die Ausgestaltungen zu den jeweiligen Vernebeleinrichtungen aus den vorstehend genannten Dokumenten in die vorliegenden Beschreibung aufgenommen werden, sowie Thermospray-Vernebler, die beispielsweise in DE 3 941 533 A1, US 4,730,111 und US 4,629,478 dargestellt werden, wobei die Ausgestaltungen zu Thermospray-Verneblern aus den jeweiligen vorstehend genannten Dokumenten in die vorliegende Beschreibung aufgenommen werden. Besonders bevorzugt sind Vernebeleinrichtungen für Verdampfungslichtstreudetektoren, beispielsweise die Vernebeleinrichtung der Fa. Sedere für den Flußbereich von 1 - 5ml/min.

Die bevorzugte Vernebeleinrichtung (2,2') der Fa. Sedere umfasst üblicherweise einen Gaseinlass (22) mit Regler zur Einstellung eines Arbeitsdrucks in der Vernebeleinrichtung (2) im Bereich von 0,5 bis 5 bar, vorzugsweise im Bereich von 1 bis 4 bar, weiter bevorzugt 2 bis 3 bar und ganz besonders bevorzugt bei 3 bar.

Zusätzlich oder alternativ umfasst der Flüssigkeitseinlass (21) der Vernebeleinrichtung (2,2') der Fa. Sedere üblicherweise einen Regler zur Einstellung der Fließgeschwindigkeit der Flüssigkeit in die Vernebeleinrichtung (2) im Bereich von 1 bis 5 ml/min, vorzugsweise, 2 bis 4 ml/min, besonders bevorzugt 3 ml/min.

Die vorstehend dargestellten bevorzugten Fließgeschwindigkeiten und Arbeitsdrücke ermöglichen eine besonders bevorzugte Vernebelung zu einem Aerosol und einen bevorzugten anschließenden Weitertransport des gebildeten Aerosols durch die erfindungsgemäße Vorrichtung (1).

In einer weiteren bevorzugten Ausgestaltung ist die erfindungsgemäße Vorrichtung (1) zusätzlich oder alternativ zu den vorstehenden bevorzugten Ausgestaltungen senkrecht zum Erdmittelpunkt aufgebaut, so dass die erste und/oder zweite Vernebeleinrichtung (2,2'), bevorzugt zweite Vernebeleinrichtung (2') am oberen Ende, d.h. an dem zum Erdmittelpunkt entferntest gelegenen Ende, der erfindungsgemäßen Vorrichtung (1), und die Kondensationskammer (5) am unteren Ende, d.h. an dem zum Erdmittelpunkt am nächsten gelegenen Ende, der erfindungsgemäßen Vorrichtung (1) angeordnet. Dieser Aufbau ist vorteilhaft, da die Schwerkraft zusätzlich zum vorliegenden Arbeitsdruck zum Weitertransport des Aerosols durch die erfindungsgemäße Vorrichtung (1) beiträgt und damit die Fließgeschwindigkeit und/oder der Arbeitsdruck verringert werden können.

In einer weiter bevorzugten erfindungsgemäßen Ausgestaltung ist die zweite Vernebeleinrichtung (2'), in welche die Referenzzubereitung eingespeist wird, am oberen Ende der Vorrichtung mit der Mischkammer (3) verbunden und die erste Vernebeleinrichtung (2), in welche die Geschmackstoffzubereitung eingespeist wird, ist unterhalb der zweiten Vernebeleinrichtung (2') mit der Mischkammer (3) verbunden. Aufgrund eines dementsprechenden Aufbaus konnte bei dem erfindungsgemäßen Verfahren zur Herstellung eines flüssigen Geschmackstoffkondensats geeignet zur sensorischen Bewertung von verstärkenden oder reduzierenden Geschmackstoffeigenschaften durch eine Testperson eine bessere Durchmischung des Aerosols der Geschmackstoffzubereitung mit dem Aerosol der Referenzzubereitung beobachtet werden. Es wird angenommen, dass für das erfindungsgemäße Verfahren zur sensorischen Bewertung aufgrund der vorstehend beschriebenen bevorzugten Ausgestaltung der erfindungsgemäßen Vorrichtung auch die Qualität der sensorischen Bewertung verbessert wird.

In einer weiteren bevorzugten Ausgestaltung ist die erfindungsgemäße Vorrichtung (1) zusätzlich oder alternativ zu den vorstehenden bevorzugten Ausgestaltungen **dadurch gekennzeichnet, dass** die Verdampfungseinrichtung (31) als Temperaturregler im Bereich von 30 bis 100 °C, vorzugsweise 50 bis 95 °C, weiter bevorzugt 70 bis 90 °C, besonders bevorzugt 75 bis 85 °C und ganz besonders von 80 °C ausgestaltet ist. Die Temperatur wird bevorzugt so eingestellt, dass eine ausreichende Menge an Ethanol in die Gasphase überführt wird, so dass der in der Separationskammer (4) aus der Vorrichtung (1) entfernte Ethanolanteil ausreicht, so dass das aus der Kondensationskammer (5) mittels des Flüssigkeitsauslasses (51) entfernte Kondensat einen Ethanolgehalt von ≤ 15 Gew.-%, bevorzugt im Bereich von 0 bis 10 Gew.-%, weiter bevorzugt im Bereich von 0,1 bis 8 Gew.-%, besonders bevorzugt im Bereich von 1 bis 5 Gew.-%, jeweils bezogen auf das Gesamtgewicht des Kondensats aufweist. Vorzugsweise liegt deshalb der Ethanolanteil, der in der Mischkammer (3) in die Gasphase übergeht, bei ≥ 50 Gew.-%, weiter bevorzugt im Bereich von 60 bis 100 Gew.-%, besonders bevorzugt im Bereich von 70 bis 95 Gew.-%, jeweils bezogen auf das Gesamtgewicht des der zugeführten Geschmackstoffzubereitung.

In einer weiteren bevorzugten Ausgestaltung ist die erfindungsgemäße Vorrichtung (1) zusätzlich oder alternativ zu den vorstehenden bevorzugten Ausgestaltungen **dadurch gekennzeichnet, dass** die Mischkammer (3) als Röhre, d.h. mit einem (im wesentlichen) kreisförmigen Querschnitt von üblicherweise 50 mm, bevorzugt 35 mm und besonders bevorzugt 25 mm und mit eine Länge von üblicherweise 150 mm, bevorzugt 120 mm und besonders bevorzugt 90 mm, ausgestaltet ist. Besonders bevorzugt weist diese Röhre einen gleichmäßigen Querschnitt auf und/oder ist nicht gekrümmt. Diese bevorzugte Ausgestaltung ist von Vorteil, da auf Grund der Röhrenform das Totvolumen minimiert wird und so zu einer turbulenzarmen Strömung führt.

In einer weiteren bevorzugten Ausgestaltung ist die erfindungsgemäße Vorrichtung (1) zusätzlich oder alternativ zu den vorstehenden bevorzugten Ausgestaltungen **dadurch gekennzeichnet, dass** die Mischkammer (3) als doppelwandige Röhre, die um ihre erste innere Wand (32) eine dazu beabstandete zweite äußere Wand (33) mit einem Einlass (34) und einem Auslass (35) für ein Mittel zur Temperaturreglung aufweist, ausgestaltet ist. In dieser bevorzugten Ausgestaltung besteht die Verdampfungseinrichtung (31) in der doppelwandigen Röhrenausgestaltung (Heizmantel), bei der das Mittel zu Temperaturregelung, üblicherweise eine Flüssigkeit, bevorzugt eine Flüssigkeit ausgewählt aus der Gruppe bestehend aus Wasser oder Mineralöl als Wärmeübertragungsmittel und Wasser, Ethylenglykol oder Mischungen aus Wasser und Ethylenglykol als Kälteübertragungsmittel, auf die gewünschte Temperatur erwärmt und durch den Einlass (34) in den Raum zwischen der ersten inneren Wand (32) und der dazu beabstandeten zweiten äußeren Wand (33) zugeführt wird und aus dem Auslass (35) gegebenenfalls entfernt wird. Diese bevorzugte Ausgestaltung ermöglicht, dass die Mischkammer (3) (im wesentlichen) konstant temperiert werden kann, da immer neues temperiertes Mittel zur Temperaturregelung dem Heizmantel zugeführt wird bzw. bereits verwendetes Mittel zur Temperaturregelung nach Austritt aus dem Auslass (35) wieder auf die Ausgangstemperatur erwärmt werden kann.

In einer alternativen Ausgestaltung kann eine erfindungsgemäß zu verwendende Verdampfungseinrichtung eine Thermoelektrische Platte (Peltier Element), ein elektrisches Heizband, ein elektrischer Heizmantel oder ein elektrisches Heizrohr sein.

In einer weiteren bevorzugten Ausgestaltung ist die erfindungsgemäße Vorrichtung (1) zusätzlich oder alternativ zu den vorstehenden bevorzugten Ausgestaltungen **dadurch gekennzeichnet, dass** die Separationskammer (4) einen mindestens gleich großen oder größeren Querschnitt, d.h. (im wesentlichen) kreisförmigen Querschnitt, im Vergleich zur Mischkammer (3) aufweist, wobei der Querschnitt der Separationskammer (4) üblicherweise im Bereich von 100 mm, bevorzugt, 70 mm, besonders bevorzugt 50 mm und die Länge der Separationskammer (4) üblicherweise im Bereich von 150 mm, bevorzugt 100 mm und besonders bevorzugt 65 mm liegt. In einer ganz besonders bevorzugten erfindungsgemäßen Ausgestaltung ist die Separationskammer (4) kugelförmig ausgestaltet. Eine Veränderung des Querschnitts führt üblicherweise zu einer Diffusion des verdampften Aerosols, so dass Strömungsturbulenzen entstehen und eine Verwirbelung in der Separationskammer (4) unterstützt wird. Hierdurch wird die Separierung des Gasanteils und des flüssigen Aerosolanteils ebenfalls unterstützt, so dass der gasförmige Ethanolanteil verbessert durch den Gasauslass (41) aus der Separationskammer und damit aus der erfindungsgemäßen Vorrichtung (1) entfernt werden kann. In einer weiteren bevorzugten Ausgestaltung wird die Verwirbelung des Aerosols in der Separationskammer (4) weiter unterstützt durch ein oder mehrere Verwirbelungseinrichtungen (42), bevorzugt stab-oder dornähnliche Einrichtungen, die mit der Separationskammerwand (43) verbunden sind und sich in das innere der Separationskammer (4) erstrecken.

In einer weiteren bevorzugten Ausgestaltung ist die erfindungsgemäße Vorrichtung (1) zusätzlich oder alternativ zu den vorstehenden bevorzugten Ausgestaltungen **dadurch gekennzeichnet, dass** der Gasauslass (41) als Röhre, d.h. mit einem (im wesentlichen) kreisförmigen Querschnitt, die durch eine äußere Wand (43) der Separationskammer (4) in einen Raum außerhalb der Vorrichtung (1) verläuft, ausgestaltet ist, wobei ein Ende der Röhre (411) innerhalb der Separationskammer (4) in einem Bereich, der gegenüber der Verbindung zur Mischkammer (3) liegt, und ein zweites Ende der Röhre (412) im Raum außerhalb der Vorrichtung (1) angeordnet ist. In einer weiteren bevorzugten Ausgestaltung ist die erfindungsgemäße Vorrichtung (1) zusätzlich oder alternativ zu den vorstehenden bevorzugten Ausgestaltungen **dadurch gekennzeichnet, dass** der Gasauslass (41) im aufgebauten Zustand am unteren Ende der Separationskammer (5) angeordnet ist und in einem Bereich oberhalb des unteren Endes durch die äußere Wand (43) der Separationskammer (4) in den Raum außerhalb der Vorrichtung (1) verläuft.

Es ist bevorzugt, dass der Gasauslass (41) am unteren Ende der Separationskammer (4), d.h. in örtlicher Nähe zur Kondensationskammer (5) angeordnet ist, da durch das Temperaturgefälle die Flüssigkeitströpfchen des Aerosols überwiegend kondensieren und durch den Flüssigkeitsauslass (51) als Kondensat abgeführt werden können, während der überwiegend separierte Gasanteil des Aerosols durch den Gasauslass (41) aus der Vorrichtung (1) entfernt werden kann.

Der Gasauslass (41) ist bevorzugt eine Glasröhre, mit einem Querschnitt von 5 bis 15 mm, weiter bevorzugt ungefähr 10 mm und ist üblicherweise weniger als 10 mm, bevorzugt weniger als 5 mm, ganz besonders bevorzugt ungefähr 3 mm oberhalb des Übergangs zur Kondensationskammer (5) angeordnet. In einer weiteren bevorzugten Ausgestaltung ist das Ende der Röhre (411) trichterförmig ausgestaltet. Durch die trichterförmige Ausgestaltung der Öffnung (411) werden Turbulenzen in der Kondensationszone vermieden, so dass das Kondensat aus dem Flüssigkeitsauslass (51) gleichmäßig tropfen kann.

In einer bevorzugten Ausgestaltung ist der Gasauslass nicht linear sondern gewinkelt, bevorzugt in einem 90° Winkel angeordnet. Hierbei sind üblicherweise die Längen des Gasauslassrohres (41) senkrecht (Senkrechtröhre) und horizontal (Querröhre) zum Erdmittepunkt ungefähr gleichlang, bevorzugt ist die Senkrechtröhre ungefähr 27 mm lang und hat einen Abstand von 3 mm zur Kondensationskammer (5) und die Querröhre ist bevorzugt 30 mm lang.

In einer weiteren bevorzugten Ausgestaltung ist die erfindungsgemäße Vorrichtung (1) zusätzlich oder alternativ zu den vorstehenden bevorzugten Ausgestaltungen **dadurch gekennzeichnet, dass** die Separationskammer (4) bei Umgebungstemperatur betrieben wird und nicht entsprechend der Kondensationskammer (5) gekühlt wird. Diese Ausgestaltung ist deshalb bevorzugt, weil durch die Kopplung der beheizten Mischkammer (3) und der gekühlten Kondensationskammer (5) über die erfindungsgemäß zu verwendende Separationskammer (4) ein "Temperaturpuffer" zwischen geschaltet wird, der Materialbeschädigungen an der Vorrichtung (1) auf Grund des Temperaturgefälles reduziert.

In einer weiteren bevorzugten Ausgestaltung ist die erfindungsgemäße Vorrichtung (1) zusätzlich oder alternativ zu den vorstehenden bevorzugten Ausgestaltungen **dadurch gekennzeichnet, dass** die Kondensationskammer (5) eine Röhre, d.h. mit (im wesentlichen) kreisförmigen Querschnitt darstellt. Wie schon zur Mischkammer (3) oben ausgeführt weist eine Röhre ein geringes Totvolumen auf.

In einer weiteren bevorzugten Ausgestaltung ist die erfindungsgemäße Vorrichtung (1) zusätzlich oder alternativ zu den vorstehenden bevorzugten Ausgestaltungen dadurch gekennzeich**net, dass** die Röhre der Kondensationskammer (5) einen im Vergleich zur Separationskammer (4) und/oder der Mischkammer (3) kleineren Querschnitt hat, d.h. üblicherweise einen Querschnitt im Bereich von 8 mm, bevorzugt 5 mm und besonders bevorzugt 2 mm und eine Länge von üblicherweise 80 mm, bevorzugt 50mm und besonders bevorzugt 35 mm aufweist. Diese Querschnittsverringerung führt zu einer Kontraktion des in der Separationskammer (4) erhaltenen und in die Kondensationskammer (5) weitertransportierten Aerosols, so dass die Wirkung der Kondensationseinrichtung (52) unterstützt wird.

In einer weiteren bevorzugten Ausgestaltung ist die erfindungsgemäße Vorrichtung (1) zusätzlich oder alternativ zu den vorstehenden bevorzugten Ausgestaltungen **dadurch gekennzeichnet, dass** die Kondensationseinrichtung (52) als Temperaturregler im Bereich von 0 bis 60 °C, vorzugsweise 5 bis 50 °C, weiter bevorzugt 10 bis 40 °C, besonders bevorzugt 15 bis 25 °C und ganz besonders von 20 °C ausgestaltet ist. Auf Grund dieser Temperaturbereiche wird die Kondensation des Aerosols zu dem erfindungsgemäßen Geschmackstoffkondensat unterstützt.

In einer weiteren bevorzugten Ausgestaltung ist die erfindungsgemäße Vorrichtung (1) zusätzlich oder alternativ zu den vorstehenden bevorzugten Ausgestaltungen **dadurch gekennzeichnet, dass** die Kondensationskammer (5) als doppelwandige Röhre, die um ihre erste innere Wand (53) eine dazu beabstandete zweite äußere Wand (54) mit einem Einlass (55) und einem Auslass (56) für ein Mittel zur Temperaturreglung, üblicherweise eine Flüssigkeit, bevorzugt eine Flüssigkeit ausgewählt aus der Gruppe bestehend aus Wasser oder Mineralöl als Wärmeübertragungsmittel und Wasser, Ethylenglykol oder Mischungen aus Wasser und Ethylenglykol als Kälteübertragungsmittel, aufweist, ausgestaltet ist. Wie bereits zur erfindungsgemäßen Mischkammer (3) oben ausgeführt, ermöglicht diese bevorzugte Ausgestaltung, dass die Kondensationskammer (5) (im wesentlichen) konstant temperiert wird.

In einer alternativen Ausgestaltung kann eine erfindungsgemäß zu verwendende Kondensationseinrichtung (52) eine Thermoelektrische Platte (Peltier Element), ein elektrisches Temperierband, ein elektrischer Temperiermantel oder ein elektrisches Temperierrohr sein.

In einer weiteren bevorzugten Ausgestaltung ist die erfindungsgemäße Vorrichtung (1) zusätzlich oder alternativ zu den vorstehenden bevorzugten Ausgestaltungen **dadurch gekennzeichnet, dass** der Flüssigkeitsauslass (51) am unteren Ende der Kondensationskammer (5) angeordnet ist. Diese bevorzugte Ausgestaltung ermöglicht, dass das gebildete Kondensat mittels der Schwerkraft durch den Flüssigkeitsauslass (51) aus der Kondensationskammer (5) und damit aus der erfindungsgemäßen Vorrichtung (1) entfernt werden kann. Zusätzlich könnten allerdings auch weitere Mittel, insbesondere Pumpen (z.B. geeignete Schlauchpumpen oder LC-Pumpen - nicht gezeigt) der Kondensationskammer (5) nachgeschaltet werden, um das Kondensat aus der Vorrichtung (1) zu entfernen.

Die erfindungsgemäße Vorrichtung (1) kann aus jedem geeigneten Material, bevorzugt aus einem oder mehreren Materialien aus der Gruppe bestehend aus Glas, Metall, Edelmetall, Stahl oder Kunststoff (z.B. Polytetrafluorethylen oder Polyetheretherketon), besonders bevorzugt Glas hergestellt werden, wobei die einzelnen Kammern und Einrichtungen so mit einander verbunden sind, so dass aus der erfindungsgemäßen Vorrichtung (1) außer an den vorgesehenen Auslassstellen, insbesondere dem Gasauslass (41) und dem Flüssigkeitsauslass (51), (im wesentlichen) weder das zugeführte Inertgas und/oder die zugeführte flüssige Zubereitung, bevorzugt die flüssige Geschmackstoffzubereitung aus der erfindungsgemäßen Vorrichtung (1) entweicht. In einer besonders bevorzugten Ausgestaltung ist die erfindungsgemäße Vorrichtung (1) zusätzlich oder alternativ zu den vorstehenden besonders bevorzugten Ausgestaltungen zum Teil oder vollständig aus wärmebeständigem Glas aufgebaut.

In einer weiteren bevorzugten Ausgestaltung kann zusätzlich oder alternativ zu den vorstehenden bevorzugten Ausgestaltungen das aus der erfindungsgemäßen Vorrichtung (1) entfernte Kondensat in zwei, drei oder mehreren Eluatfraktionen separiert werden, wobei die Fraktionen besonders bevorzugt derart separiert werden, dass eine Fraktion im wesentlichen einen Geschmackstoff und einen Referenzstoff umfasst. Zur Ermittlung einer Geschmackstoffkonzentratfraktion, die einen Geschmackstoff und einen Referenzstoff enthält, können ein oder mehrere, gleiche oder unterschiedliche geeignete Detektoren (nicht gezeigt), bevorzugt ausgewählt aus der Gruppe bestehend aus Massenselektiven Detektoren (MS), Ultraviolett Detektoren (UV), Diodenarray Detektoren (DAD), Brechungsindex-Detektoren (RI), Verdampfungslichtstreu Detektoren (ELSD - evaporative light scattering detector), Fluoreszenz Detektoren (FLD) oder Charged Aerosol Detektoren (CAD), die vor und/oder nach der erfindungsgemäßen Vorrichtung (1) angeordnet sind, verwendet werden. Diese bevorzugte Ausgestaltung ermöglicht die sensorische Bewertung hinsichtlich der verstärkenden oder maskierenden Eigenschaften des isolierten Geschmackstoffes gegenüber dem sensorischen Eindruck des Referenzstoffes alleine.

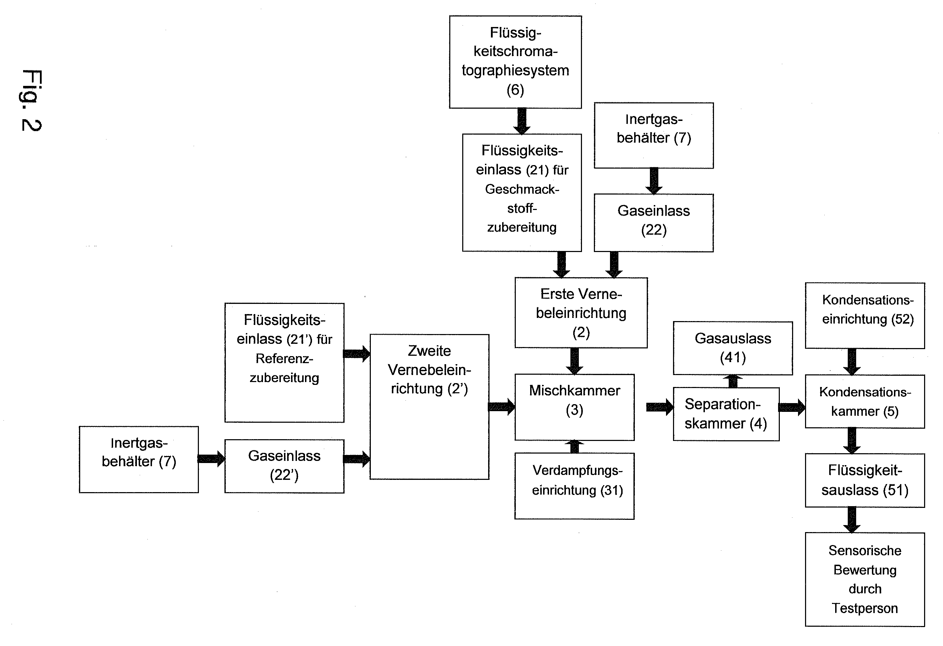

**Figur 2** zeigt einen schematischen Aufbau einer erfindungsgemäßen Vorrichtung (1), die ebenfalls die vorstehend dargestellten bevorzugten Ausgestaltungen in allen denkbaren Kombinationen umfasst, gekoppelt an ein erfindungsgemäß zu verwendendes Flüssigchromatographiesystem (6).

Die bevorzugte Ausgestaltung der erfindungsgemäße Vorrichtung (1) kann in einer bevorzugten Ausgestaltung mit den erfindungsgemäß verwendbaren Flüssigkeitschromatographiesystemen (6) unterschiedlicher Separationstechniken direkt, d.h. über den Flüssigkeitseinlass (21), verbunden werden, so dass das Eluat durch den Flüssigkeitseinlass (21) in die erste Vernebeleinrichtung (2) zugeführt werden kann.

Die Koppelung der erfindungsgemäßen Vorrichtung (1) ist zum einen deshalb bevorzugt, weil durch die flüssigchromatographische Auftrennung einer flüssigen Geschmackstoffprobe, die zwei, drei oder mehrere Geschmackstoffe umfasst, bereits eine zeitliche Auftrennung der Geschmackstoffe durch die eingesetzten Elutionsmittel Wasser und Ethanol, gegebenenfalls im Gradientenbetrieb vorgenommen wird, wobei das Eluat aus einer flüssigchromatographischen Auftrennung der erfindungsgemäßen Geschmackstoffzubereitung entspricht. Zum anderen umfasst das erfindungsgemäß zu verwendende Flüssigkeitschromatographiesystem (6) bereits eine Detektoreinrichtung die der erfindungsgemäßen Vorrichtung (1) dementsprechend vorgeschaltet ist, so dass eine bessere Abschätzung ermöglicht wird, wann der oder die Geschmackstoffe nach Durchlauf durch die erfindungsgemäße Vorrichtung (1) in dem hergestellten Geschmackstoffkondensats zu erwarten sind.

In einer alternativen bevorzugten Ausgestaltung ist die erfindungsgemäße Vorrichtung (1) indirekt mit einem üblicherweise verwendbaren, vorstehend genannten Flüssigchromatographiesystem (6) gekoppelt, d.h. dass ein oder mehrere mittels dieses erfindungsgemäß zu verwendenden Flüssigkeitschromatographiesystems (6) separierte Eluatfraktionen, die bevorzugt jeweils nur einen Geschmackstoff enthalten, separat mit der erfindungsgemäßen Vorrichtung (1) nacheinander über den Flüssigkeitseinlass (21) in die erste Vernebeleinrichtung (2) zugeführt werden.

Durch die kompakte Bauform der erfindungsgemäßen Vorrichtung (1) und durch die zusätzliche Zuführung der Referenzzubereitung und/oder des Inertgases in die zweite Vernebeleinrichtung (2') kann die Fließgeschwindigkeit eines LC-Eluats als flüssige Geschmackstoffzubereitung bzw. die Fließgeschwindigkeit des Aerosols, d.h. die Dauer des Transports durch die erfindungsgemäße Vorrichtung (1), so eingestellt werden, dass die flüssigchromatographische Trennleistung gewährleistet bleibt und so eine zeitnahe ("on-line") bzw. synchrone sensorische Bewertung hinsichtlich des Geschmackes eines einzigen Geschmackstoffes und damit des sensorischen Beitrags des Geschmackstoffes durch eine Testperson ermöglicht wird. Üblicherweise beträgt bei einem Druck von 3 bar an der Vernebeleinrichtung (2) die Aerosol/Gasgeschwindigkeit ∼65ml/s. Die Passagedauer des Aerosols variiert in Abhängigkeit zu den Temperaturen (Verdampfung/Kondensation) in der Vorrichtung (1), zwischen Bruchteilen von Sekunden bis 1, 2 oder wenige Sekunden.

Dementsprechend kann gemäß einer weiteren bevorzugten Ausgestaltung zusätzlich oder alternativ zu den vorstehenden bevorzugten Ausgestaltungen das erfindungsgemäße Verfahren zur Herstellung eines flüssigen Geschmackstoffkondensats geeignet zur sensorischen Bewertung von verstärkenden oder reduzierenden Geschmackstoffeigenschaften durch eine Testperson dadurch gekennzeichnet sein, dass die Geschmackstoffzubereitung, die in Schritt a) der Vernebeleinrichtung (2) zugeführt wird, ein mittels eines erfindungsgemäß zu verwendenden Flüssigchromatographiesystems (6) gewonnenes Eluat ist.

In einer weiteren bevorzugten Ausgestaltung kann zusätzlich oder alternativ zu den vorstehenden bevorzugten Ausgestaltungen das erfindungsgemäße Verfahren zur Herstellung eines flüssigen Geschmackstoffkondensats geeignet zur sensorischen Bewertung von verstärkenden oder reduzierenden Geschmackstoffeigenschaften durch eine Testperson dadurch gekennzeichnet sein, dass das Flüssigchromatographiesystem (6) mit dem Flüssigkeitseinlass (21) der ersten Vernebeleinrichtung (2) so verbunden ist, dass das Eluat als die Geschmackstoffzubereitung direkt nach Auftrennung mittels des Flüssigchromatographiesystems (6) durch den Flüssigkeitseinlass (21) in die Vernebeleinrichtung (2) zugeführt wird.

In einer weiteren bevorzugten Ausgestaltung kann zusätzlich oder alternativ zu den vorstehenden bevorzugten Ausgestaltungen das erfindungsgemäße Verfahren zur Herstellung eines flüssigen Geschmackstoffkondensats geeignet zur sensorischen Bewertung von verstärkenden oder reduzierenden Geschmackstoffeigenschaften durch eine Testperson dadurch gekennzeichnet sein, dass das Eluat aus dem Flüssigchromatographiesystems (6) mittels einer Detektoreinrichtung (8), die zwischen das Flüssigchromatographiesystem (6) und der ersten Vernebeleinrichtung (2) angeordnet ist, auf das Vorhandensein eines Geschmackstoffes in dem Eluat untersucht wird.

In einer weiteren bevorzugten Ausgestaltung kann zusätzlich oder alternativ zu den vorstehenden bevorzugten Ausgestaltungen das erfindungsgemäße Verfahren zur Herstellung eines flüssigen Geschmackstoffkondensats geeignet zur sensorischen Bewertung von verstärkenden oder reduzierenden Geschmackstoffeigenschaften durch eine Testperson dadurch gekennzeichnet sein, dass die Referenzzubereitung und das Inertgas in Schritt d) mindestens über einen Zeitraum, innerhalb dem mittels der Detektoreinrichtung (8) ein Geschmackstoff in dem Eluat detektiert wurde, der zweiten Vernebeleinrichtung (2') zugeführt wird.

In einer weiteren bevorzugten Ausgestaltung kann zusätzlich oder alternativ zu den vorstehenden bevorzugten Ausgestaltungen das erfindungsgemäße Verfahren zur Herstellung eines flüssigen Geschmackstoffkondensats geeignet zur sensorischen Bewertung von verstärkenden oder reduzierenden Geschmackstoffeigenschaften durch eine Testperson dadurch gekennzeichnet sein, dass in Schritt c) eine Geschmackstoffzubereitung mit einem Ethanolgehalt von > 15 Gew.-% bezogen auf das Gesamtgewicht der Geschmackstoffzubereitung der ersten Vernebeleinrichtung (2) zugeführt wird und in Schritt f) soviel gasförmiges Ethanol aus der Separationskammer (4) entfernt wird, dass das in Schritt h) entfernte flüssige Geschmackstoffkondensat einen Ethanolgehalt von ≤ 15 Gew.-% bezogen auf das Gesamtgewicht des Geschmackstoffkondensats aufweist.

In einer weiteren bevorzugten Ausgestaltung kann zusätzlich oder alternativ zu den vorstehenden bevorzugten Ausgestaltungen das erfindungsgemäße Verfahren zur Herstellung eines flüssigen Geschmackstoffkondensats geeignet zur sensorischen Bewertung von verstärkenden oder reduzierenden Geschmackstoffeigenschaften durch eine Testperson dadurch gekennzeichnet sein, dass in Schritt c) eine Geschmackstoffzubereitung mit einem Ethanolgehalt von > 15 Gew.-% bezogen auf das Gesamtgewicht der Geschmackstoffzubereitung der ersten Vernebeleinrichtung (2) zugeführt wird und in Schritt d) eine Referenzzubereitung ohne Ethanol oder mit einem ausreichend geringen Ethanolanteil der zweiten Vernebeleinrichtung (2') zugeführt wird, so dass das in Schritt h) entfernte flüssige Geschmackstoffkondensat einen Ethanolgehalt von ≤ 15 Gew.-% bezogen auf das Gesamtgewicht des Geschmackstoffkondensats aufweist.

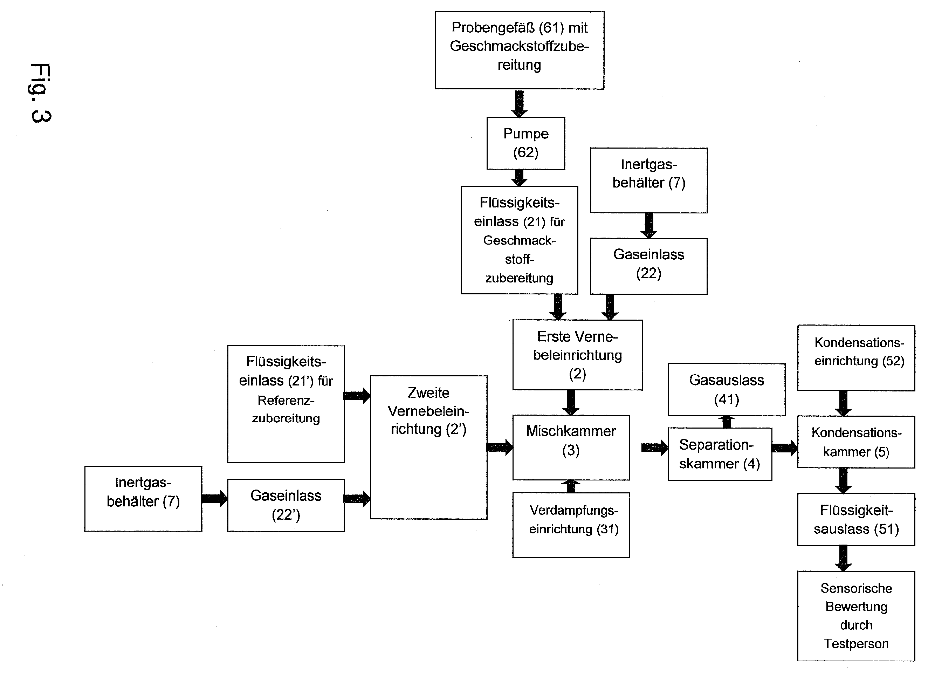

**Figur 3** zeigt einen schematischen Aufbau einer erfindungsgemäßen Vorrichtung (1) , die ebenfalls die vorstehend dargestellten bevorzugten Ausgestaltungen in allen denkbaren Kombinationen umfasst, gekoppelt an ein Probengefäß (61) enthaltend eine erfindungsgemäße Geschmackstoffzubereitung mit bevorzugt einem Geschmackstoff, die so miteinander verbunden sind, dass diese Geschmackstoffzubereitung gegebenenfalls mittels einer geeigneten üblichen Pumpe (62), beispielsweise LC-Pumpe oder Schlauchpumpe, durch den Flüssigkeitseinlass (21) in die erste Vernebeleinrichtung (2) zugeführt wird. Zwischen dem Probengefäß (61) und dem Flüssigkeitseinlass (21) kann bevorzugt eine Detektoreinrichtung (8) zwischengeschaltet sein, um besser beurteilen zu können, wann der oder die Geschmackstoffe durch die erfindungsgemäße Vorrichtung (1) durchgeleitet werden.

Üblicherweise werden die Detektoren ausgewählt aus der Gruppe bestehend aus Massenselektiven Detektoren (MS), Ultraviolett Detektoren (UV), Diodenarray Detektoren (DAD), Brechungsindex-Detektoren (RI), Verdampfungslichtstreu Detektoren (ELSD - evaporative light scattering detector), Fluoreszenz Detektoren (FLD) oder Charged Aerosol Detektoren (CAD).

### Ausführungsbeispiel:

Die vorliegende Erfindung wird im Folgenden durch ein Ausführungsbeispiel beschrieben, das jedoch den Schutzumfang der erfindungsgemäßen Gegenstände nicht limitiert.

### Beispiel 1: Identifizierung von bittermaskierende Stoffen aus Herba Santa (Eriodictyon californicum)

### 1. Herstellung einer Probelösung

Eine Probelösung Herba Santa wurde durch Lösung/Suspension von 500mg Herba Santa in einem Gemisch Wasser/Ethanol (1:1 / v:v), mit anschließender Membranfiltration des nicht gelösten Rückstandes, hergestellt.

### 2. Durchführung einer Flüssigchromatographie der Probelösung

Die verwendete HTLC- Vorrichtung, bestehend aus einem Degasser (3315, Fa. ERC), zwei Pumpen (Sun Flow 100, Fa. Sunchrom), einem Mischer (dynamic/static Mixer, Fa. Sunchrom), einem Autosampler (Midas, Fa. Spark), einem Säulenofen (Polaratherm, Fa. Selerity) und einem Diodenarray-Detektor (Spectra Flow 600, Fa. Sunchrom) wurde mit einer Hamilton PRP-1 250x10mm Säule ausgestattet und mit einem Fluss von 3 ml/min eines Elutionsmittels, bestehend aus 30 Gew.-% Ethanol und 70 Gew.-% Wasser, konditioniert.

Zur Probenaufgabe in die verwendete, konditionierte HTLC-Vorrichtung wurden mit dem Autosampler 100µl der hergestellten klaren Lösung injiziert.

Nach erfolgter Konditionierung erfolgt die chromatographische Analyse nach folgendem Programm:

| Zeit [min] | Fluss [ml/min ] | Wasser [%] | Ethanol [%] | Elutionsgradient [%/min] | Temperatur Säulenofen [°C] | Temperaturgradient [°C/min) | Diode array detector (DAD) |
|---|---|---|---|---|---|---|---|
| 0 | 3 | 7 0 | 30 | 1,4 | 120 | isotherm | 210 nm |
| 50 | 3 | 0 | 100 | Isokrat isch | 120 | isotherm | 210 nm |
| 65 | 3 | 0 | 100 | Isokrat isch | 120 | isotherm | 210 nm |

Die Änderungen von Temperatur, Fluss und Elutionsmittel-Zusammensetzung während des Programms erfolgen linear. Die in Eriodictyon californicum vorhandene Verbindung Homoeriodictyol eluierte nach chromatographischer Auftrennung bei einer Ethanolkonzentration von 45%.

Das Eluat wird im Anschluss an die chromatographische Trennung unter folgenden Bedingungen über den Flüssigkeitseinlass (21) direkt in die Vernebeleinrichtung (2) erfindungsgemäßen Vorrichtung (1) eingeleitet:

| Gasdruck im Gaseinlass (22) | Gasdruck im Gaseinlass (22') | Fluss im Flüssigkeitseinlass (21) | Fluss im Flüssigkeitseinlass (21') | Temperatur Verdampfungseinricht ung (31) | Temperatur Kondensationseinrichtung (52) |
|---|---|---|---|---|---|
| 3 bar | 3 bar | 3 ml/min | 3 ml/min | 80°C | 30°C |

Über den Flüssigkeitseinlass (21) wird die 0,10 Gew.-%ige Coffeinlösung (1000 ppm) als Referenzzubereitung und Stickstoff als Inertgas der zweiten Vernebeleinrichtung (2) zugeführt synchron zum Eluat als Geschmackstoffzubereitung in die Mischkammer (3) eingebracht, um die Vermischung des sensorisch zu bewertenden Geschmackstoffes und der Referenzzubereitung (Coffeinlösung) herbeizuführen und um eine Reduzierung des Ethanolgehaltes zu ermöglichen.

Das am Ende im Flüssigkeitsausgang (51) der Kondensationskammer (5) gewonnene Geschmackstoffkondensat wies einen Ethanolgehalt von ca. ~6% auf.
Das gewonnene Kondensat wurde direkt durch ein Panel aus trainierten Testpersonen sensorisch bewertet.

Um eine Blindprobe zu erhalten wurde der obige Versuch mit reinem Wasser anstelle der Geschmackstoffzubereitung wie oben beschrieben wiederholt. Das so gewonnene Kondensat wird im Vergleich mit dem zuvor gewonnenen Geschmackstoffkondensat verkostet, um eine Abnahme der Bitterkeit auf Grund des Geschmackstoffes in der Geschmackstoffzubereitung feststellen zu können.

## Patentansprüche

1. Vorrichtung (1) zur Herstellung eines flüssigen Geschmackstoffkondensats geeignet zur sensorischen Bewertung durch eine Testperson mit zwei Vernebeleinrichtungen (2, 2'), einer Mischkammer (3), einer Separationskammer (4) und einer Kondensationskammer (5),
**dadurch gekennzeichnet, dass**
- die erste Vernebeleinrichtung (2) einen Flüssigkeitseinlass (21), einen Gaseinlass (22) und eine Verbindung zur Mischkammer (3) umfasst oder daraus besteht, so dass eine flüssige Geschmackstoffzubereitung umfassend ein oder mehrere Geschmackstoffe, Wasser und/oder Ethanol mittels des Flüssigkeitseinlasses (21) und ein Inertgas mittels des Gaseinlasses (22) in die Vernebeleinrichtung (2) zuführbar sind und die Vernebeleinrichtung (2) geeignet ist, die Flüssigkeit und das Inertgas zu einem Aerosol zu vernebeln und so in die Mischkammer (3) zuzuführen,
- die zweite Vernebeleinrichtung (2') einen Flüssigkeitseinlass (21'), einen Gaseinlass (22') und eine Verbindung zur Mischkammer (3) umfasst oder daraus besteht, so dass eine flüssige Referenzzubereitung umfassend ein oder mehrere Referenzstoffe, Wasser und/oder Ethanol mittels des Flüssigkeitseinlasses (21') und ein Inertgas mittels des Gaseinlasses (22') in die Vernebeleinrichtung (2') zuführbar sind und die Vernebeleinrichtung (2') geeignet ist, die Flüssigkeit und das Inertgas zu einem Aerosol zu vernebeln und so in die Mischkammer (3) zuzuführen,
- die Mischkammer (3) eine Verdampfungseinrichtung (31) und eine Verbindung zur Separationskammer (4) umfasst oder daraus besteht, wobei die Mischkammer (3) nach der ersten und zweiten Vernebeleinrichtung (2,2') angeordnet und so damit verbunden ist, dass die mittels der ersten und zweiten Vernebeleinrichtung (2,2') gebildeten Aerosole in die Mischkammer (3) separat zuführbar sind und die Verdampfungseinrichtung (31) geeignet ist, einen Teil des oder das Ethanol der zugeführten Aerosole teilweise oder vollständig in die Gasphase zu überführen,
- die Separationskammer (4) einen Gasauslass (41) und eine Verbindung zur Kondensationskammer (5) umfasst oder daraus besteht, wobei die Separationskammer (4) nach der Mischkammer (3) angeordnet und so damit verbunden ist, dass das in die Gasphase überführte Ethanol und das verbleibende Aerosol in die Separationskammer (4) zuführbar sind und ein Teil oder die Ethanol-Gasphase aus der Vorrichtung (1) durch den Gasauslass (41) entfernbar ist und
- die Kondensationskammer (5) einen Flüssigkeitsauslass (51) und eine Kondensationseinrichtung (52) umfasst oder daraus besteht, wobei die Kondensationskammer (5) nach der Separationskammer (4) angeordnet und so damit verbunden ist, dass das in der Separationskammer (4) nach teilweiser oder vollständiger Entfernung der Ethanol-Gasphase verbleibende Aerosol in die Kondensationskammer (5) zuführbar ist und die Kondensationseinrichtung (5) geeignet ist, das zugeführte Aerosol ganz oder teilweise zu kondensieren und das Kondensat aus der Kondensationskammer (5) durch den Flüssigkeitsauslass (51) entfernbar ist.

2. Vorrichtung gemäß Anspruch 1, **dadurch gekennzeichnet, dass** die erste und/oder zweite Vernebeleinrichtung (2,2') bei einer Temperatur ≤ 75 °C, vorzugsweise in einem Temperaturbereich von 10 bis 40 °C, weiter bevorzugt in einem Temperaturbereich von 15 bis 30 °C und ganz besonders bevorzugt in einem Temperaturbereich von 20 bis 25 °C betrieben wird.

3. Vorrichtung gemäß Anspruch 1 oder 2, **dadurch gekennzeichnet, dass** der Gaseinlass (22,22') der ersten und/oder zweiten Vernebeleinrichtung (2,2') einen Regler zur Einstellung eines Arbeitsdrucks in der Vernebeleinrichtung (2,2') im Bereich von 0,5 bis 5 bar, vorzugsweise im Bereich von 1 bis 4 bar, weiter bevorzugt 2 bis 3 bar und ganz besonders bevorzugt bei 3 bar umfasst.

4. Vorrichtung gemäß einem der vorstehenden Ansprüche, **dadurch gekennzeichnet, dass** der Flüssigkeitseinlass (21,21') der ersten und/oder zweiten Vernebeleinrichtung (2,2') einen Regler zur Einstellung der Zufuhrgeschwindigkeit der Flüssigkeit in die Vernebeleinrichtung (2,2') im Bereich von 1 bis 5 ml/min, vorzugsweise, 2 bis 4 ml/min, besonders bevorzugt 3 ml/min umfasst.

5. Vorrichtung gemäß einem der vorstehenden Ansprüche, **dadurch gekennzeichnet, dass** ein Flüssigchromatographiesystem (6) mit dem Flüssigkeitseinlass (21) der ersten Vernebeleinrichtung (2) verbunden ist, so dass ein mittels des Flüssigchromatographiesystems (6) gewonnenes Eluat als die Geschmackstoffzubereitung durch den Flüssigkeitseinlass (21) der Vernebeleinrichtung (2) zuführbar ist.

6. Vorrichtung gemäß Anspruch 5, **dadurch gekennzeichnet, dass** zwischen dem Flüssigchromatographiesystem (6) und der ersten Vernebeleinrichtung (2) eine Detektoreinrichtung (8) zur Detektion des oder der Geschmackstoffe in dem Eluat angeordnet ist.

7. Verfahren zur Herstellung eines flüssigen Geschmackstoffkondensats geeignet zur sensorischen Bewertung von verstärkenden oder reduzierenden Geschmackstoffeigenschaften durch eine Testperson umfassend oder bestehend aus den folgenden Schritten:
a) Bereitstellung einer flüssigen Geschmackstoffzubereitung, die ein oder mehrere Geschmackstoffe, Wasser und/oder Ethanol umfasst oder daraus besteht, sowie eines Inertgases,
b) Bereitstellung einer flüssigen Referenzzubereitung, die ein oder mehrere Referenzstoffe, Wasser und/oder Ethanol umfasst oder daraus besteht, sowie eines Inertgases,
c) Zuführung der flüssigen Geschmackstoffzubereitung aus Schritt a) durch einen Flüssigkeitseinlass (21) und des Inertgases durch einen Gaseinlass (22) in eine erste Vernebeleinrichtung (2) mittels der die Geschmackstoffzubereitung und das Inertgas zu einem Aerosol vernebelt und so einer Mischkammer (3) zugeführt werden,
d) Zuführung der flüssigen Referenzzubereitung aus Schritt b) durch einen Flüssigkeitseinlass (21') und des Inertgases durch einen Gaseinlass (22') in eine zweite Vernebeleinrichtung (2') mittels der die Referenzzubereitung und das Inertgas zu einem Aerosol vernebelt und so einer Mischkammer (3) zugeführt werden,
e) Überführung eines Teils des oder des gesamten Ethanolanteils der in Schritt d) in die Mischkammer (3) zugeführten Aerosole mittels einer Verdampfungseinrichtung (31) in eine Gasphase,
f) Weiterleitung des in Schritt e) gebildeten Aerosols in eine mit der Mischkammer (3) verbundenen Separationskammer (4) mit Gasauslass (41) und Entfernung eines Teils des oder des gesamten gasförmigen Ethanolanteils aus der Separationskammer (4) durch den Gasauslass (41),
g) Weiterleitung des in Schritt f) gebildeten Aerosols in eine mit der Separationskammer (4) verbundene Kondensationskammer (5) mit Flüssigkeitsauslass (51) und Kondensationseinrichtung (52) und Kondensation des Aerosols mittels der Kondensationseinrichtung (52) zu einem Geschmackstoffkondensat umfassend oder bestehend aus dem oder den Geschmackstoffen, dem oder den Referenzstoffen, Wasser und/oder Ethanol und
h) Entfernung des in Schritt g) gebildeten Geschmackstoffkondensats aus der Kondensationskammer (5) durch den Flüssigkeitsauslass (51).

8. Verfahren gemäß Anspruch 7, **dadurch gekennzeichnet, dass** die Geschmackstoffzubereitung, die in Schritt a) der ersten Vernebeleinrichtung (2) zugeführt wird, ein mittels eines Flüssigchromatographiesystems (6) gewonnenes Eluat ist.

9. Verfahren gemäß Anspruch 7 oder 8, **dadurch gekennzeichnet, dass** das Eluat aus dem Flüssigchromatographiesystems (6) mittels einer Detektoreinrichtung (8), die zwischen das Flüssigchromatographiesystem (6) und der ersten Vernebeleinrichtung (2) angeordnet ist, auf das Vorhandensein eines Geschmackstoffes in dem Eluat untersucht wird.

10. Verfahren gemäß einem der Ansprüche 7 bis 9, **dadurch gekennzeichnet, dass** in Schritt c) eine Geschmackstoffzubereitung mit einem Ethanolgehalt von > 15 Gew.-% bezogen auf das Gesamtgewicht der Geschmackstoffzubereitung der ersten Vernebeleinrichtung (2) zugeführt wird und in Schritt d) eine Referenzzubereitung ohne Ethanol oder mit einem ausreichend geringen Ethanolanteil der zweiten Vernebeleinrichtung (2') zugeführt wird, so dass das in Schritt h) entfernte flüssige Geschmackstoffkondensat einen Ethanolgehalt von ≤ 15 Gew.-% bezogen auf das Gesamtgewicht des Geschmackstoffkondensats aufweist.

11. Verfahren zur sensorischen Bewertung von verstärkenden oder reduzierenden Geschmackeigenschaften eines oder mehrerer Geschmackstoffe umfassend oder bestehend aus den folgenden Schritten:
i. Herstellung eines flüssigen Geschmackstoffkondensates umfassend oder bestehend aus ein oder mehreren Geschmackstoffen, ein oder mehreren Referenzstoffen, Wasser und/oder Ethanol gemäß einem der Verfahren nach Ansprüchen 7 bis 11 und
ii. Zuführung des Geschmackstoffkondensates aus Schritt i) in den Mund einer Testperson zur sensorischen Bewertung von verstärkenden oder reduzierenden Geschmackeigenschaften.

12. Verwendung einer Vorrichtung gemäß einem der Ansprüche 1 bis 6 zur Herstellung eines flüssigen Geschmackstoffkondensates umfassend oder bestehend aus ein oder mehreren Geschmackstoffen, ein oder mehreren Referenzstoffen, Wasser und/oder Ethanol.

13. Verfahren zur Verdünnung einer flüssigen Geschmackstoffzubereitung umfassend oder bestehend aus den folgenden Schritten:
a) Bereitstellung einer flüssigen Geschmackstoffzubereitung, die ein oder mehrere Geschmackstoffe, Wasser und Ethanol mit einem Ethanolgehalt von > 15 Gew.-% bezogen auf das Gesamtgewicht der Geschmackstoffzubereitung umfasst oder daraus besteht, sowie eines Inertgases,
b) Bereitstellung einer Verdünnungslösung, die Wasser und/oder Ethanol umfasst oder daraus besteht, wobei der Ethanolgehalt ≤ 15 Gew.-% bezogen auf das Gesamtgewicht der Lösung beträgt, sowie eines Inertgases,
c) Zuführung der flüssigen Geschmackstoffzubereitung aus Schritt a) durch einen Flüssigkeitseinlass (21) und des Inertgases durch einen Gaseinlass (22) in eine erste Vernebeleinrichtung (2) mittels der die Geschmackstoffzubereitung und das Inertgas zu einem Aerosol vernebelt und so einer Mischkammer (3) zugeführt werden,
d) Zuführung der Verdünnungslösung aus Schritt b) durch einen Flüssigkeitseinlass (21') und des Inertgases durch einen Gaseinlass (22') in eine zweite Vernebeleinrichtung (2') mittels der die Verdünnungslösung und das Inertgas zu einem Aerosol vernebelt und so der Mischkammer (3) zugeführt werden,
e) Überführung eines Teils des oder des gesamten Ethanolanteils der in Schritt d) in die Mischkammer (3) zugeführten Aerosole mittels einer Verdampfungseinrichtung (31) in eine Gasphase,
f) Weiterleitung des in Schritt e) gebildeten Aerosols in eine mit der Mischkammer (3) verbundenen Separationskammer (4) mit Gasauslass (41) und Entfernung eines Teils des oder des gesamten gasförmigen Ethanolanteils aus der Separationskammer (4) durch den Gasauslass (41),
g) Weiterleitung des in Schritt f) gebildeten Aerosols in eine mit der Separationskammer (4) verbundene Kondensationskammer (5) mit Flüssigkeitsauslass (51) und Kondensationseinrichtung (52) und Kondensation des Aerosols mittels der Kondensationseinrichtung (52) zu einem Geschmackstoffkondensat umfassend oder bestehend aus dem oder den Geschmackstoffen, Wasser und Ethanol, wobei der Ethanolgehalt ≤ 15 Gew.-% bezogen auf das Gesamtgewicht des Kondensates beträgt und
h) Entfernung des in Schritt g) gebildeten Geschmackstoffkondensats aus der Kondensationskammer (5) durch den Flüssigkeitsauslass (51).

14. Verfahren zur sensorischen Bewertung eines oder mehrerer Geschmackstoffe umfassend oder bestehend aus den folgenden Schritten:
iii. Herstellung eines flüssigen Geschmackstoffkondensats umfassend oder bestehend aus ein oder mehreren Geschmackstoffen, Wasser und Ethanol gemäß Anspruch 13, wobei der Ethanolgehalt ≤ 15 Gew.-% bezogen auf das Gesamtgewicht des Kondensates beträgt, und
iv. Zuführung des Geschmackstoffkondensates aus Schritt i) in den Mund einer Testperson zur sensorischen Bewertung des oder der Geschmackstoffe.

15. Verwendung einer Vorrichtung gemäß einem der Ansprüche 1 bis 6 zur Herstellung eines flüssigen Geschmackstoffkondensates umfassend oder bestehend aus ein oder mehreren Geschmackstoffen, Wasser und Ethanol, wobei der Ethanolgehalt ≤ 15 Gew.-% bezogen auf das Gesamtgewicht des Kondensates beträgt.
